# EUROPEAN PATENT APPLICATION

(11) **EP 4 382 534 A1**
(43) Date of publication of application: **12.06.2024**
(21) Application number: 22306796.8
(22) Date of filing: 06.12.2022
(51) Int. Cl.: C07K 14/47, C12Q 1/6851

(54) **SYNTHETIC PEPTIDE FOR USE IN AN IMMUNOMOLECULAR ASSAY**

(71) Applicant: BIOMÉRIEUX, 69280 Marcy L'Etoile (FR)
(72) Inventor: BEDIN, Frédéric, 69007 LYON (FR); BENOIT, Vincent, 69009 LYON (FR)
(74) Representative: Plasseraud IP

(57) **Abstract**

The invention relates to the field of immunoassay, in particular, of immunomolecular assay. The invention provides an internal control for use in immunomolecular assay such as proximity extension assay or proximity ligation assay. Such internal control comprises a synthetic peptide of the following formula (I)

R₁-Z-R₂ (I)

wherein
- Z is a spacer consisting of 1 to 3 unnatural amino acids,
- R₁ and R₂ are two different peptide moieties, wherein a first peptide moiety of R₁ or R₂ comprises a Lys tag and a second peptide moiety comprises a His tag.

## Description

### FIELD OF THE INVENTION

The invention relates to the field of immunoassay, in particular, of immunomolecular assay. The invention provides an internal control for use in immunomolecular assay such as proximity extension assay or proximity ligation assay.

### BACKGROUND

There is a continuing need to develop simple, minimally invasive tools for disease screening, diagnosis and prevention. The PLA (Proximity Ligation Assay) and, more recently, the PEA (Proximity Extension Assay) can be a suitable answer to these needs. PEA and PLA are homogeneous dual-recognition immunoassays, which have proven to be sensitive, specific and convenient for the detection or quantitation of one or more biological analytes [1] [2]. Proximity assays rely on the principle of proximity probing, wherein an analyte is detected by the binding of at least two specific probes, which, when brought in close proximity one to the other upon binding to the analyte, allow a nucleic acid-based signal to be generated and possibly quantified [3]. Due to the characteristics of these assays, the native conformation of the antigen to be detected is preserved.

One of the main limitations of proximity extension assay embodiments described in the literature is assay time, which is quite long (more than 90 min, qPCR excluded) due to multiple dilution and incubation steps in different buffers.

It was previously demonstrated that it was possible to dramatically reduce the time to result of proximity extension assay to make it more compatible with clinical diagnostics requirements (unpublished results). The model analyte chosen for that demonstration was procalcitonin, an example of a biomarker for which a reduction in assay time translates to better patient management. Procalcitonin is a small polypeptide whose blood level rises and can be routinely measured early and specifically in the case of bacterial infection. Procalcitonin helps to determine infection severity, to monitor its evolution and response to treatment, and to adapt the treatment. It is generally accepted that for values below 0.25 µg/L a bacterial infection is excluded. A plasma dose above 0.5 µg/L indicates local infection, or even systemic bacterial infection for a dose above 2.0 µg/L. At a dose of 10 µg/L or more, a diagnosis of severe sepsis and/or septic shock can be made [4-7].

One key benefit of proximity extension assay over other more conventional, nonhomogeneous immunoassay techniques (such as sandwich ELISA) is that proximity extension assay requires neither a solid phase nor washes, which makes for a simpler, easier to implement assay protocol. Nevertheless, proximity extension assay still involves multiple reagent addition, dilution and incubation steps, which can be prone to human error (when performed manually) or system malfunction (when performed in an automated manner). In particular, unintended or accidental deviation from the correct protocol can lead to an incorrect qPCR signal, which can be erroneously interpreted. In order to mitigate this risk, a fail-safe internal control (IC) was developed. The role of this control is to serve as an indicator of any malfunction occurring over the course the assay, from sample addition to qPCR, so that the result can be flagged as invalid.

One basic requirement of this control is that it should always yield the same level of signal (when the proximity extension assay protocol is correctly executed), regardless of the particular sample being tested and the analyte concentration in the sample.

In the present disclosure, a synthetic peptide made up of two artificial, non-human sequences separated by a neutral peptide spacer was generated and a proof of concept for its use as a fail-safe internal control in an immunomolecular assay was provided by implementing the internal control in a proximity extension assay for procalcitonin detection in human patient samples.

### SUMMARY

A first object of the disclosure relates to a synthetic peptide of the following formula (I)

R₁-Z-R₂ (I)

wherein
Z is a spacer consisting of 1 to 3 unnatural amino acids,
R₁ and R₂ are different peptide moieties, wherein one peptide moiety of R1 or R2 comprises a polyhistidine tag (or His tag) and the other peptide moiety comprises a polylysine tag (or Lys tag).

Another object of the disclosure relates to a composition comprising the synthetic peptide as herein defined, in solution in water, in the presence of a buffer or in a biological fluid.

Another object of the disclosure relates to a kit for use as an internal control or quantitative calibrator in an immunomolecular assay, such as a proximity extension assay, a proximity extension or ligation assay, or an immunoPCR, said kit comprising at least the following components:
(i) a synthetic peptide as disclosed herein, and,
(ii)at least two proximity probes, each proximity probe comprising an antigen-binding moiety coupled to a nucleic acid domain,
wherein a first proximity probe comprises an anti-His tag antibody or its antigen-binding fragment thereof that binds specifically to a His tag, and, a second proximity probe comprises an anti-Lys tag antibody or its antigen-binding fragment thereof that binds specifically to a Lys tag.

In specific embodiments, the immunomolecular assay is performed with a plasma or blood sample, for example a blood sample obtained from a human subject.

The disclosure also relates to the use of the synthetic peptide as disclosed herein, or a kit comprising such synthetic peptide, as an internal control or quantitative calibrator in an immunoassay, preferably in a proximity extension assay.

Another object of the present disclosure is a method for detecting or quantifying an analyte by proximity extension assay, said method comprising the use of a synthetic peptide as herein disclosed as an internal control or quantitative calibrator.

### DETAILED DESCRIPTION

### The synthetic peptide

A compound according to the present disclosure is a a synthetic peptide of the following formula (I)

R₁-Z-R₂ (I)

wherein
Z is a spacer consisting of 1 to 3 unnatural amino acids,
R₁ and R₂ are different peptide moieties, wherein one peptide moiety of R₁ or R₂ comprises a Lys tag and the other peptide moiety comprises a His tag.

Such synthetic peptide having both a Lys tag and a His tag is biepitopic. As used herein, a biepitopic compound is a compound which comprises two distinct epitopes which can be recognized specifically by two distinct specific monoclonal antibodies. In the present disclosure, the synthetic peptide is recognized by a monoclonal antibody binding specifically to the Lys tag and a monoclonal antibody binding specifically to the His tag. In particular, there is no substantial steric hindrance which would prevent simultaneous binding of both antibodies to their respective epitopes of the synthetic peptide.

The peptide of the present disclosure is a synthetic peptide, i.e. a peptide which does not exist in nature, the Lys tag and His tag being artificial sequence which do not exist in nature.

The synthetic peptide according to the present description is advantageously adapted for long term storage. Indeed, peptides are typically kept at -20°C. However, it was demonstrated that synthetic peptides presented in this disclosure are stable at least twelve weeks (86 days) at 4°C, and at least 24 h at room temperature. It is therefore possible to use these synthetic peptides under common storage conditions and over a prolonged period of time with limited risk of significant degradation.

As used herein, the term His tag refers to a peptide sequence which comprises from 4 to 8 histidine residues (polyhistidine peptide) and which can be recognized specifically by anti-His tag antibodies, such as 18D12 [10]. A preferred embodiment of a His tag is the 6xHistidine tag of SEQ ID NO:1 (MRGSHHHHHH), and more particularly the 6xHistidine tag of SEQ ID NO:2 (MRGSHHHHHHSVDES).

As used herein, the term Lys tag refers to a peptide sequence which comprises from 4 to 8 lysine residues (polylysine peptide) and which can be recognized specifically by anti-Lys tag antibodies, such as 2G4A12 or 5F12E4 [10]. A preferred embodiment of a Lys tag is the 6xLys tag of SEQ ID NO:3 (GKKKKKKSV), and more particularly the 6xLys tag of SEQ ID NO:4 (GKKKKKKSVDESL). In the context of the present description, antibodies 2G4A12 and 5F12E4 can also be named 2G4 or 5F12 respectively.

The spacer Z is there to provide an appropriate spacing between the two epitope tags. The inventors have indeed determined that an appropriate length between about 20 to about 30 Angstroms of the spacer Z is optimal for use of the synthetic peptide as an internal control or quantitative calibrator in a proximity extension assay.

The spacer essentially consists of 1 to 3 unnatural amino acids, preferably the spacer is a dipeptide or a tripeptide.

As used herein, the term "unnatural amino acid" refers to amino acids which are not found in natural polypeptide chain. Such unnatural amino acid may be found as secondary metabolites in bacterial, fungi, plants or marine organisms, or may be synthesized chemically.

In a preferred embodiment, said unnatural amino acid has the following formula:
-NH-(Z')-COO-, wherein Z' is a linear saturated or unsaturated hydrocarbonated chain optionally comprising one or more heteroatom, and said chain comprises between 6 and 20 atoms other than hydrogen.

In a preferred embodiment, Z' is a saturated or unsaturated hydrocarbonated chain comprising one or more heteroatom, wherein the heteroatom is selected from O, N and S.

In specific embodiments, said unnatural amino acid is 8-amino-2,4-dioxa octanoic acid.

In a particularly preferred embodiment, Z is a dipeptide or tripeptide of 8-amino-2,4-dioxa octanoic acid.

In another embodiment, Z is the polyethylene glycol (PEG) of formula H-(O-CH2-CH2)ₙ-OH, wherein n is comprised from 1 to 4, and preferably n is equal to 2.

The spacer is flanked at its N-terminal end and C-terminal end by the peptide moieties of R₁ and R₂ respectively.

In addition to the Lys tag or His tag, the peptide moeities of R₁ and R₂ may further comprise 1, 2, 3, 4 or 5 additional amino acids adjacent at the N-terminal end or C-terminal end provided that such additional amino acid do not affect the simultaneous binding of the anti-His tag and anti-Lys tag antibody respectively.

Accordingly, in a specific embodiment, R₁ is a peptide of the following sequence:
X₋₁₀X₋₉X₋₈X₋₇(X₋₆)ₙ X₋₅X₋₄X₋₃X₋₂X₋₁, wherein:
- X₋₁₀ is optional or a non-polar amino acid, preferably a methionine,
- X₋₉ is optional or a basic amino acid, preferably an arginine,
- X_$ is optional or a non-polar amino acid, preferably a glycine,
- X₋₇ is optional or a polar amino acid, preferably a serine,
- X₋₆ is a histidine or lysine,
- X₋₅ is optional or a polar amino acid, preferably a serine,
- X₋₄ is optional or non-polar amino acid, preferably a valine,
- X₋₃ is optional or an acidic amino acid, preferably an aspartic acid,
- X₋₂ is optional or an acidic amino acid, preferably a glutamic acid,
- X₋₁ is optional or a polar amino acid, preferably a serine, and
- n is an integer between 4 to 8, preferably equal to 6.

In more specific embodiment, R₁ essentially consists of one of the following sequences: MRGSHHHHHH (SEQ ID NO: 1) or MRGSHHHHHHSVDES (SEQ ID NO:2).

In other specific embodiment which may be combined with the previous embodiment, R₂ is a peptide of the following sequence:
X₊₁(X₊₂)ₚX₊₃X₊₄X₊₅X₊₆X₊₇X₊₈ wherein:
- X₊₁ is optional or a non-polar amino acid, preferably a glycine,
- X₊₂ is a lysine or histidine,
- X₊₃ is optional or a polar amino acid, preferably a serine,
- X₊₄ is optional or a non-polar amino acid, preferably a valine,
- X₊₅ is optional or an acidic amino acid, preferably an aspartic acid,
- X₊₆ is optional or an acidic amino acid, preferably a glutamic acid,
- X₊₇ is optional or a polar amino acid, preferably a serine,
- X₊₈ is optional or non-polar amino acid, preferably a leucine, and
- p is an integer between 4 to 8, preferably equal to 6.

In more specific embodiment R₂ essentially consists of one of the following sequences: GKKKKKKSV (SEQ ID NO:3) or GKKKKKKSVDESL (SEQ ID NO:4).

As used herein, the term "non-polar amino acids" refer to amino acids in which the variable R-group is comprised of mostly hydrocarbons, and for methionine, also a sulphur atom, and which do not have any polar properties. In specific embodiments for R₁ and R₂ residues, non-polar amino acids are selected from the group consisting of glycine, alanine, valine, leucine, isoleucine, proline, phenylalanine, methionine and tryptophane.

As used herein, the term "polar amino acids" refer to hydrophilic amino acids, either negatively charged, or positively charged, or uncharged. all polar amino acids have either an OH or NH₂ group (when in aqueous environment) and can therefore form hydrogen bonds with other suitable groups. In specific embodiments for R₁ and R₂ residues, polar amino acids are selected from the group consisting of serine, cysteine, threonine, tyrosine, asparagine and glutamine.

As used herein, the term "basic amino acids" refer to amino acids having basic side chains at neutral pH. In specific embodiments for R₁ and R₂ residues, basic amino acids are selected from the group consisting of arginine, histidine and lysine.

As used herein, the term "acidic amino acid" refers to amino acid having acids carboxylic acid on its side chain that gives it acidic (proton-donating) properties. In specific embodiments for R₁ and R₂ residues, acidic amino acids are selected from aspartic acid and glutamic acid.

In a preferred embodiment, the synthetic peptide has the following sequence: MRGSHHHHHH-(N)m-GKKKKKKSV, wherein N is an unnatural amino acid with a side chain having a length of about 10 to 30 angstroms and m is 1, 2, or 3. In specific embodiments, N is an unnatural amino acid having the following formula:
-NH-(Z')-COO-, wherein Z' is a linear saturated or unsaturated hydrocarbonated chain optionally comprising one or more heteroatom, and said chain comprises between 6 and 20 atoms other than hydrogen.

In a more preferred embodiment, the synthetic peptide has the following sequence: MRGSHHHHHH-(N)ₘ-GKKKKKKSV, wherein N is 8-amino-2,4-dioxa octanoic acid and m is 2 (SEQ ID NO:5) or 3 (SEQ ID NO:6).

In a preferred embodiment, the synthetic peptide has the following sequence: MRGSHHHHHHSVDES-(N)m-GKKKKKKSVDESL, wherein N is an unnatural amino acid with a side chain having a length of about 10 to 30 angstroms and m is 1, 2, or 3. In specific embodiments, N is an unnatural amino acid having the following formula:
-NH-(Z')-COO-, wherein Z' is a linear saturated or unsaturated hydrocarbonated chain optionally comprising one or more heteroatom, and said chain comprises between 6 and 20 atoms other than hydrogen.

In another preferred embodiment, the synthetic peptide has the following sequence: MRGSHHHHHHSVDES-(N)m-GKKKKKKSVDESL, wherein N is 8-amino-2,4-dioxa octanoic acid and m is 2 (SEQ ID NO:7) or 3 (SEQ ID NO:8).

### The composition and kits of the disclosure

The synthetic peptide of the present disclosure, in particular for its use as an internal control or quantitative calibrator in a proximity extension assay, is comprised in an aqueous composition, optionally further comprising a buffering agent.

In a preferred embodiment, the synthetic peptide is comprised in a sterile aqueous composition and advantageously stored at -20°C.

Buffering agents for use in the composition comprising the synthetic peptide of the disclosure are well-known in the art and for example selected from the group consisting of phosphate-buffered saline (PBS), HEPES and Tris-HCl.

In particular embodiments, the composition is a concentrated solution for dilution in a reaction solution for carrying out a proximity extension assay. A concentrated solution may comprises an amount which twice, three times, four times, five times, ten times, twenty times or more, the final concentration of the synthetic peptide to be used as an internal control in a proximity extension assay. Typically, the synthetic peptide may be present at a concentration between 1 pg/mL to 1 µg/mL.

The composition may also comprise other compounds including, salts, proteins (e.g. Bovine Serum Albumin), synthetic polymers, such as dextran or polyethylene glycol, or surfactants.

The composition is particularly useful as an internal control when used in combination with a pair proximity probes including anti-Lys Tag antibody and anti-His Tag antibody, as disclosed in the next sections.

Hence, the disclosure also relates to a kit for use as an internal control or quantitative calibrator in a proximity extension assay, said kit comprising at least the following components:
- a synthetic peptide as described in the previous section, preferably the synthetic peptide of the following sequence: MRGSHHHHHH-(N)m-GKKKKKKSV, or of the following sequence MRGSHHHHHHSVDES(N)m-GKKKKKKSVDESL
   wherein N is 8-amino-2,4-dioxa octanoic acid and m is 2 or 3, and for example the synthetic peptide of SEQ ID NO:6 or SEQ ID NO:8,
- at least two proximity probes, each proximity probe comprising an antigen-binding moiety coupled to a nucleic acid domain, wherein a first proximity probe comprises an anti-His tag antibody or its antigen-binding fragment thereof that binds specifically to a His tag, and, a second proximity probe comprising an anti-Lys tag antibody or its antigen-binding fragment thereof that binds specifically to a Lys tag.

In a particular embodiment, the synthetic peptide is chosen from the synthetic peptides of SEQ ID NO:7 or SEQ ID NO:8.

Anti-His and anti-Lys tag antibodies are well known by the skilled person in the art and any available commercial references are suitable for use in the kit according to the present description. Additionally, the skilled person using the hybridomas technique is able to produce monoclonal antibodies that specifically binds to one of the sequences of R₁ and R₂ peptide moieties.

As used herein, the term "proximity probe" refers to probes which can be used in a proximity extension assay. A proximity extension assay refers to any assay for detecting or quantifying an analyte in a sample and which relies on the principle of "proximity probing", i.e. wherein an analyte (or an internal control such as the synthetic peptide) is detected by the binding of multiple (i.e. two or more, generally two or three) proximity probes, which when brought into proximity by binding to the analyte allow a signal to be generated.

Typically, each proximity probe comprises a nucleic acid domain (or moiety, e.g. oligonucleotide) linked to the antigen-binding domain (or moiety, e.g. an antibody), and generation of the signal involves an interaction between the nucleic acid moieties (such as forming a duplex by hybridization of complementary strands). In other words, the signal generation is dependent on the interaction of the proximity probes.

The term "hybridization" or "hybridizes" as used herein refers to the formation of a duplex between nucleotide sequences which are sufficiently complementary to form duplexes via Watson-Crick base pairing. Two nucleotide sequences are "complementary" to one another when those molecules share base pair organization homology. "Complementary" nucleotide sequences will combine with specificity to form a stable duplex under appropriate hybridization conditions.

For instance, two sequences are complementary when a section of a first sequence can bind to a section of a second sequence in an anti-parallel sense wherein the 3'- end of each sequence binds to the 5'-end of the other sequence and each A, T(U), G and C of one sequence is then aligned with a T(U), A, C and G, respectively, of the other sequence. Two sequences need not have perfect homology to be "complementary". Usually, two sequences are sufficiently complementary when at least about 85% (preferably at least about 90%, and most preferably at least about 95%) of the nucleotides share base pair organization over a defined length of the molecule or the domains that are determined to be complementary. The nucleic acid domains of the first and second proximity probes thus contain a region of complementarity for the nucleic acid domain the other proximity probe.

The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies. Accordingly, an anti-His tag antibody is an antibody (or a fragment thereof) which specifically binds to polyhistidine tag. An anti-Lys tag antibody is an antibody (or a fragment thereof) which specifically binds to a polylysine tag.

In natural antibodies of rodents and primates, two heavy chains are linked to each other by disulfide bonds, and each heavy chain is linked to a light chain by a disulfide bond. There are two types of light chains, lambda (λ) and kappa (κ). There are five main heavy chain classes (or isotypes) which determine the functional activity of an antibody molecule: IgM, IgD, IgG, IgA and IgE. Each chain contains distinct sequence domains. In typical IgG antibodies, the light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR).

The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) can participate in the antibody binding site, or influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, typically includes six CDRs, comprising the CDRs set from each of a heavy and a light chain V region. Framework Regions (FRs) refer to amino acid sequences interposed between CDRs. Accordingly, the variable regions of the light and heavy chains typically comprise 4 framework regions and 3 CDRs of the following sequence: FR1-CDR1-FR2-CDR2-FR3-CDR3-FR4.

The residues in antibody variable domains are conventionally numbered according to a system devised by Kabat et al. This system is set forth in Kabat et al., 1987, in Sequences of Proteins of Immunological Interest, US Department of Health and Human Services, NIH, USA (Kabat et al., 1992, hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system.

In specific embodiments, an antibody provided herein is an antibody fragment, and more particularly any protein including an antigen-binding domain of an antibody as disclosed herein. Antibody fragments include, but are not limited to, Fv, Fab, F(ab')2, Fab', dsFv, scFv, sc(Fv)2 and diabodies.

The term "monoclonal antibody" or "monoclonal antibody composition" as used herein refers to a preparation of antibody molecules of single molecular composition. A monoclonal antibody composition displays a single binding specificity and affinity for a particular epitope.

The phrases "an antibody recognizing an antigen" and "an antibody having specificity for an antigen" are used interchangeably herein with the term "an antibody which binds specifically to an antigen".

Specificity can further be exhibited by, e.g., an about 10:1, about 20:1, about 50:1, about 100:1, 10.000:1 or greater ratio of affinity/avidity in binding to the specific antigen versus nonspecific binding to other irrelevant molecules (in this case the specific antigen is a His-tag or Lys-tag peptide sequences).

The term "affinity", as used herein, means the strength of the binding of an antibody to an epitope.

As used herein, an antibody which binds specifically to an antigen (e.g. His tag or Lys tag) is an antibody which binds to its antigen with a K_{D} of at least 100nM, for example at least 10nM, or at least 1nM or less, as measured by surface plasmonic resonance (SPR) assay, such as Biacore assay.

In specific embodiments, the anti-His tag antibody directed to a 6xHistine tag (Ladavière et al; Bioconjug Chem. 1998;9(6):655-61. Epub 1998/11/17. doi: 10.1021/bc970208i. PubMed PMID: 9815157) comprises at least the heavy chain variable region (VH) and light chain variable region (VL) of the following monoclonal antibodies called 18D12. Such antibodies are for example the monoclonal antibody named "clone 4D11" provided by Merck (ref 05-531), the monoclonal antibody named "6x-His Tag Monoclonal antibody (HIS.H8)" provided by ThermoFisher (ref MA1-21315) or the antibody named "clone AD1.1.10" provided by R&S Systems (ref MAB050).

In specific embodiments, the anti-Lys tag antibody directed to a 6xLysine tag (Ladavière et al; Bioconjug Chem. 1998;9(6):655-61. Epub 1998/11/17. doi: 10.1021/bc970208i. PubMed PMID: 9815157) comprises at least the heavy chain variable region (VH) and light chain variable region (VL) of the following monoclonal antibodies called 2G4A12 or 5F12E4 (preferably 2G4A12).

In preferred embodiments, said anti-His tag antibody for use in the kit is the antibody 18D12.

In preferred embodiments, said anti-Lys tag antibody for use in the kit is the antibody 2G4A12 or 5F12E4, more preferably 2G4A12.

In a more preferred embodiment of the kit, the anti-His tag is the antibody 18D12 and the anti-Lys tag is the antibody 2G4A12.

In specific embodiments, the kit comprises at least
- a first proximity probe comprising an anti-His tag antibody, such as 18D12 or its antigen-binding region, coupled to a first oligonucleotide,
- a second proximity probe comprising an anti-Lys tag antibody, such as 2G4A12 or 5F12E4 or its antigen-binding region, coupled to a second oligonucleotide.

The oligonucleotides may be conjugated to the analyte-binding domain, either directly or through a linking group. The components can be covalently bonded to one another through functional groups, as is known in the art, where such functional groups may be present on the components or introduced onto the components using one or more steps, e.g. oxidation reactions, reduction reactions, cleavage reactions and the like. Functional groups that may be used in covalently bonding the components together to produce the proximity probe include: hydroxy, sulfhydryl, amino, and the like. The particular portion of the different components that are modified to provide for covalent linkage may be chosen so as not to substantially adversely interfere with that component's desired binding affinity for the target analyte. Where necessary and/or desired, certain moieties on the components may be protected using blocking groups, as is known in the art, see e.g. Green & Wuts, Protective Groups in Organic Synthesis (John Wley & Sons) (1991). Methods for producing nucleic acid/antibody conjugates are well known to those of skill in the art. See e.g. U.S. Patent No. 5,733,523, the disclosure of which is herein incorporated by reference.

In other embodiments, proximity probes may be produced using in vitro protocols that yield nucleic acid-protein conjugates, i.e. molecules having nucleic acids, e.g. coding sequences, covalently bonded to a protein, i.e. where the analyte-binding domain is produced in vitro from vectors which encode the proximity probe. Examples of such in vitro protocols of interest include: RepA based protocols (see e.g., Fitzgerald, Drug Discov. Today (2000) 5:253-258 and WO 98/37186), ribosome display based protocols (see e.g., Hanes et al., Proc. Natl Acad. Sci. USA (1997) 94:4937-42; Roberts, Curr Opin Chem Biol (1999) Jun; 3: 268-73; Schaffitzel et al., J Immunol Methods (1999) Dec 10; 231 : 1 19-35; and WO 98/54312), etc.

In specific embodiments, the first and second oligonucleotides coupled to the anti-His tag and anti-Lys tag antibodies respectively are designed so that they can hybridize when the antibodies bind to the synthetic peptide of the present disclosure (and preferably the synthetic peptide of SEQ ID NO:6 or SEQ ID NO:8).

Typically, said first and second oligonucleotides have between 29 and 56 bases. In one embodiment of the kit as disclosed herein, one oligonucleotide has 29 bases and the other has 31 bases. In another embodiment of the kit as disclosed herein, one oligonucleotide has 49 bases and the other has 56 bases.

In specific embodiments, the regions of complementarity (i.e. hybridisation regions) in the oligonucleotides for use in the kit may have a length in the range of 4-30 bases e.g. 6-20, 6-18, 7-15, 8-12, or 9-11 bases.

In preferred embodiments, the pair of oligonucleotides have the following complementary sequences of GACGACTTC and GAAGTCGTC respectively.

In a preferred embodiment, a kit of the present disclosure comprises, in addition the synthetic peptide as disclosed above, at least
(i) a first proximity probe comprising a anti-His tag antibody conjugated to an oligonucleotide of SEQ ID NO:9 and
(ii) a second proximity probe comprising a anti-Lys tag antibody conjugated with an oligonucleotide of SEQ ID NO:10.

In a more preferred embodiment, a kit of the present disclosure comprises, in addition the synthetic peptide as disclosed above, at least
(i) a first proximity probe comprising 8D12 anti-His tag antibody conjugated to an oligonucleotide of SEQ ID NO:9, and
(ii) a second proximity probe comprising 2G4 anti-Lys tag antibody conjugated with an oligonucleotide of SEQ ID NO:10.

In an even more preferred embodiment, a kit of the present disclosure comprises at least
(i) a synthetic peptide of SEQ ID NO:6 or SEQ ID NO:8,
(ii) a first proximity probe comprising 8D12 anti-His tag antibody conjugated to an oligonucleotide of SEQ ID NO:9, and
(iii) a second proximity probe comprising 2G4 anti-Lys tag antibody conjugated with an oligonucleotide of SEQ ID NO:10.

### Methods of use of the synthetic peptide

The synthetic peptide, or its composition, or kit comprising such synthetic peptide, as described above, are advantageously used as an internal control or quantitative calibrator in an immunoassay, and preferably in an immunomolecular assay such as a proximity extension assay, a proximity ligation assay or an immuno-PCR, for detecting and/or quantifying an analyte in a sample.

As used herein, the term "analyte" refers to any substance (e.g. molecule) or entity which is desired to be detected by an immunoassay such as a proximity extension assay. The analyte is the "target" of the proximity extension assay. The analyte may accordingly be any biomolecule or chemical compound it may be desired to detect, for example a peptide or protein, or nucleic acid molecule or a small molecule, including organic and inorganic molecules. The analyte may be a cell or a microorganism, including a virus, or a fragment or product thereof. All that is required is that the analyte is capable of simultaneously binding at least two binding partners (more particularly, the analyte- binding domains of at least two proximity probes). In preferred embodiments, the analyte is a protein or a polypeptide and any molecule which includes a protein or polypeptide component. For example, the analyte is a protein which may be found in blood sample, typically blood sample.

As used herein, the term "internal control" more specifically refers to an indicator of any malfunction occurring over the course of the assay, so that the result can be flagged as invalid. In preferred embodiments, the internal control will yield about the same (expected) result irrespective of the particular sample being tested and the analyte concentration in the sample.

In such situation, the internal control may also be used as a quantitative calibrator: The concentration of the internal control is known, and the signal obtained is also known. Accordingly, the various signals obtained with analyte detected, for example in multiplexed proximity extension assay carried out with the internal control, may be adjusted as compared to the signal obtained with the internal control.

As used herein, the proximity extension assay is a dual-recognition immunoassay, wherein two matched antibodies conjugated to unique nucleic acid domains (herein called proximity probes) simultaneously bind to an analyte in solution. This brings the two antibodies into proximity, allowing their nucleic acid domains to hybridize, serving as template for a DNA-polymerase-dependent extension step. This creates a double-stranded DNA "barcode" which is unique for the specific antigen and quantitatively proportional to the initial concentration of target protein or analyte. The hybridization and extension are immediately followed by PCR amplification. The resulting DNA amplicon can then be detected and/or quantified. Methods using proximity extension assays have been described in Lundberg et al [1], Fredriksson et al [2], and Greenwood et al [3]. Other variation of proximity probe-based assays have been described in the art. For example, proximity extension assays are described in WO01/61037, US6,511,809, WO03/055231, WO2005/123963, and WO2006/137932.

In a particular embodiment, the internal control is used in a method for detecting and/or quantifying an analyte in a sample, which comprises at least the following steps:
(a) contacting said sample with at least one set of at least a first and second proximity probes, which proximity probes each comprises an analyte-binding domain (e.g. antigen-binding domain) and a nucleic acid domain (e.g. oligonucleotide) and can simultaneously bind to the analyte,
(b) allowing the nucleic acid domains of the proximity probes to interact with each other upon binding of said proximity probes to said analyte, wherein said interaction comprises the formation of a duplex,
(c) detecting and/or quantifying the formation of the duplex, typically by extension and PCR amplification based on such duplex.

As used herein, the term "duplex" refers to the hybridization of two complementary nucleic acid domains of the proximity probes.

Step (c) may further comprise the following sub-steps of
(c1) extending the 3' end of at least one nucleic acid domain of said duplex to generate an extension product,
(c2) amplifying and detecting the extension product.

The term "amplifying" or "amplified" is used generally herein to include any means of increasing the number of copies of an extension product, or part thereof, in the assay as a means of signalling the presence of the target analyte in the sample.

For instance, any amplification means known in the art may be used in the methods of the disclosure, e.g. Primer Chain Reaction (PCR), Ligand Chain Reaction (LCR), etc.

It will be apparent that it is not necessary to amplify all of an extension product in order to determine whether the sample comprises the target analyte. It is necessary to amplify only a portion of the extension product that was not present in the sample before the extension reaction occurred. For example, the extension product will effectively comprise two parts: a first "old" part (the existing part) containing the nucleotide sequence that made up the nucleic acid domain of the proximity probe and a second "new" part (the extended part) containing the nucleotide sequence generated by the templated extension reaction. In specific embodiments, it is the detection of the second "new" or "extended" part that may allow the detection of the of the target analyte, i.e. if there is no analyte, there will be no extension and hence no "new" or "extended" part.

Thus, in a preferred aspect of the disclosure, the step of amplifying the extension product comprises amplifying a portion of the extended part of the extension product. A portion of the extended part need be of sufficient size that it can be distinguished from other sequences present in the sample. In effect, the portion of the extended part of the extension product acts as a unique identifier or signal that corresponds to the presence of the target analyte. Hence, if the portion comprises a nucleotide sequence that is not otherwise present in the sample, the amplification of this sequence is sufficient to signal the presence and quantity of the target analyte in the sample.

Thus, the portion may comprise at least 8 nucleotides, preferably at least 9, 10, 11, 12, 13, 14, 15, 16, 17, 18, 19, 20 nucleotides. Portions of the extended part of the extension product may generally be in the range of between from about 8 to about 90 nucleotides in length, from about 12 to about 70 nucleotides in length, from about 14 to about 50 nucleotides in length, from about 16 to about 40 nucleotides in length.

Whilst it is envisaged that the whole of the extension product may be amplified, i.e. both the existing and extended parts, it is sufficient that the amplification product comprises at least a portion of the extended part of the extension product. In one aspect of the disclosure, primers may be designed to flank either side of the portion of the extended part of the extension product and amplification of that portion, e.g. by PCR, and the amplification product (comprising the portion of the extended product) may be detected as described below.

Examples of PCR amplification, DNA polymerase and 3'exonuclease for use in the proximity extension assays are also described in WO2012104261 and WO2013113699.

Typically, real-time PCR (RT-PCR) or quantitative PCR (qPCR) may be used.

In a particularly preferred embodiment, the extension product is amplified by PCR, wherein the PCR is quantitative PCR and the amplified nucleic acid molecules are quantified using an intercalating dye. In a preferred embodiment the intercalating dye is selected from SYBR Green^{®} and EvaGreen^{™}.

Hence, the disclosure also relates to a method for detecting and/or quantifying an analyte in a sample, typically a biological sample, said method comprising:
(i) at least one proximity extension assay by incubating the test sample with at least a pair of proximity probes directed to the analyte to be detected,
(ii) at least one control test of the validity of the proximity assay by incubating the test sample with the synthetic peptide as disclosed herein (for example, the synthetic peptide of SEQ ID NO:6) and a pair of corresponding proximity probes comprising anti-His tag and anti-Lys tag antibodies as provided with the kit of the present disclosure.

As used herein, the term "biological sample" refers to a fluid sample that contains an analyte from biological origin, for example from viruses and other microorganisms, plants, animals.

For example, said biological sample may be obtained from urine, blood including without limitation peripheral blood or plasma or serum, stool, sputum, saliva, bronchoalveolar fluid, endotracheal aspirates, wounds, cerebrospinal fluid, lymph node, exudate and more generally any human biopsy tissue or body fluids, tissues or materials. In a more specific embodiment, the biological sample is a whole blood sample.

In more specific embodiments, the analyte to be detected is human procalcitonin.

The biological sample may be treated or not prior to its use in the proximity extension assay.

The proximity extension assay of step (i) and the control test of step (ii) may be carried out either simultaneously or sequentially, on either the same solid support or in separate solid support.

In specific embodiments, they are performed within the same biological sample in a multiplexed proximity extension assay.

Methods for making multiplexed proximity extension assays are for example described by Assarsson et al [20].

In addition to use as an internal positive control, the synthetic peptide or kit of the present disclosure may also be used as a quantitative calibrator in a sample test.

Accordingly, the disclosure relates to a method for quantifying an analyte in a test sample, said method comprising:
(i) at least one proximity extension assay by incubating the test sample with at least a pair of proximity probes directed to the analyte to be detected,
(ii) at least one control test of the validity of the proximity assay by incubating the test sample with the synthetic peptide as disclosed herein (for example, the synthetic peptide of SEQ ID NO:6) and a pair of corresponding proximity probes comprising anti-His tag and anti-Lys tag antibodies as provided with the kit of the present disclosure,
(iii) determining the quantity of the analyte in the test sample by comparing the signal obtained at step (i) with the signal obtained at step (ii).

Steps (i) and (ii) can be performed simultaneously within the same test sample, using multiplexed proximity extension assay.

For determining the quantity of the analyte at step (iii), the signal obtained at step (ii) with the proximity probes for quantifying the internal control (i.e. the synthetic peptide of the disclosure, for example a peptide of SEQ ID NO:6) is quantified to obtain a control value. In parallel, the signal obtained at step (i) with the proximity probes for detecting the analyte is quantified to obtain a test value. The relative quantification is obtained by determining the ratio between the test value and the control value. Absolute quantification may be obtained if the absolute quantity of the internal control in the test sample is known.

In specific embodiments, for quantification of both signals, a DNA amplicon representative of the internal control and another DNA amplicon representative of the analyte may be quantified, in a multiplexed proximity extension assay, typically using qPCR or RT-PCR.

The invention will now be further illustrated by the following figures and examples. However, these examples and figures should not be interpreted in any way as limiting the scope of the present invention.

### BRIEF DESCRIPTION OF THE FIGURES

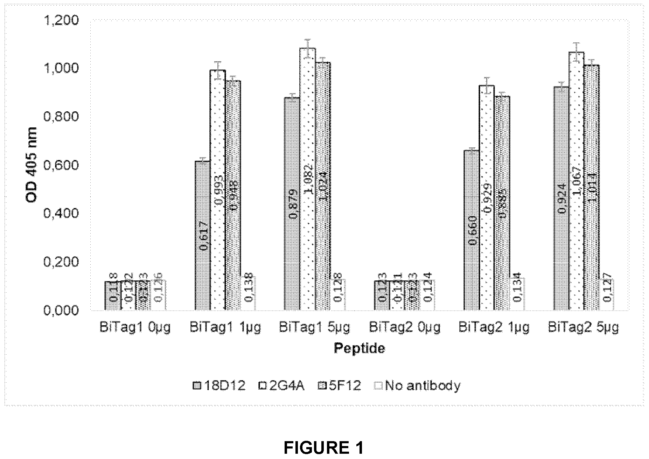

**Figure 1****:** Optical density (OD) at 405 nm for the different tag antibodies tested by ELISA for different concentrations of Bitag 1 or Bitag 2 peptides (0, 1 and 5 µg/mL). 18D12 corresponds to the His-tag monoclonal antibody, 2G4 and 5F12 correspond to the Lys-tag monoclonal antibodies. The results correspond to the mean of three experiments conducted in duplicate for each condition.

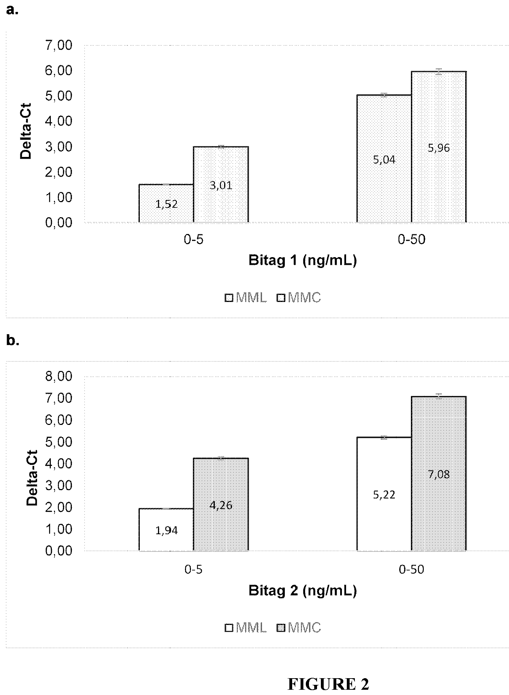

**Figure 2****:** Evaluation of different peptides (a : Bitag 1 ; b : Bitag 2) with MML and MMC Proximity probes. Results are given in Delta-Ct, corresponding to the difference between the Ct value for 0 ng/mL of Bitag and the Ct value for other concentrations of Bitag (5 and 50 ng/mL, respectively). Ct corresponds to cycle threshold, i.e., the smallest qPCR cycle number at which the fluorescence signal is higher than the background. The results correspond to the mean of three duplicated experiments.

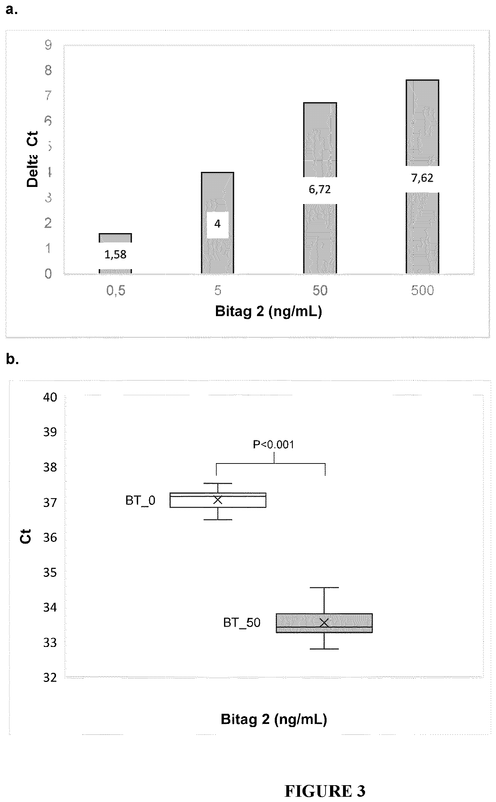

**Figure 3**: (a) Proximity extension assay in human plasma spiked with different concentrations of Bitag 2 peptide. Results are given in Delta-Ct0-X (with X = 0.5, 5, 50 and 500 ng/mL, respectively) and correspond to the mean of three duplicated experiments. (b) Box plots of Proximity extension assay in human plasmas spiked with either 0 (BT_0) or 50 ng/mL (BT_50) of Bitag 2 peptide. Results are given in Ct values and were obtained from 13 (BT_0) and 28 (BT_50) plasma specimens. Each assay was carried out in duplicate. (x): mean value.

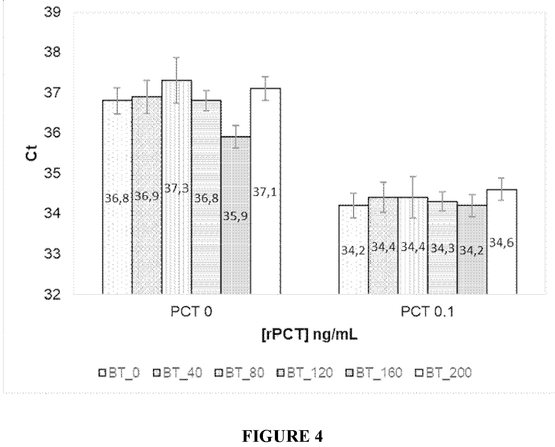

**Figure 4****:** Procalcitonin proximity extension assay on PBS-BSA buffer spiked with recombinant procalcitonin (rPCT) (0 and 0.1 ng/mL) and peptide Bitag 2 (BT) at different concentrations (from 0 to 200 ng/mL). The results, given in Ct, corresponded to the mean of three duplicated experiments.

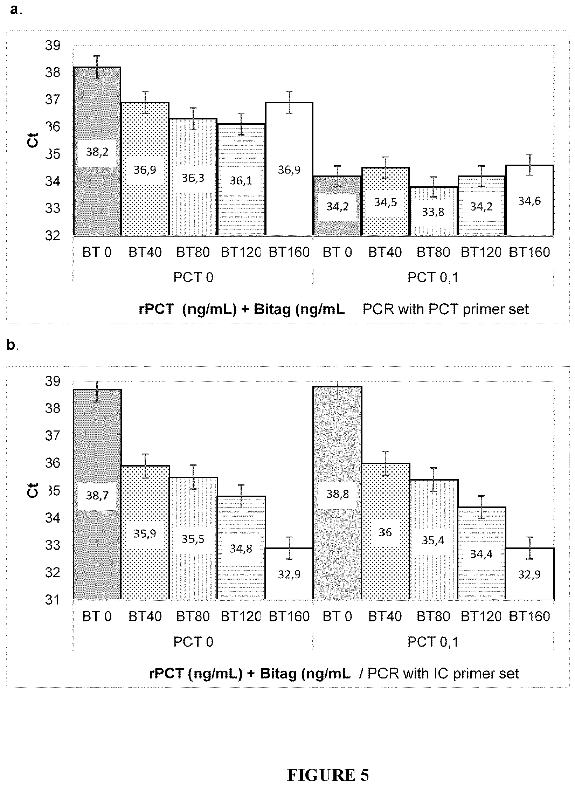

**Figure 5****:** Procalcitonin and Internal Control Proximity extension assay on PBS-BSA buffer spiked with rPCT (0 and 0.1 ng/mL) and peptide Bitag 2 (BT) at different concentrations (from 0 to 160 ng/mL). After PEA, PCR amplification is either conducted, (a) with PCR primers specific of the PCT PEA probes or, (b) with PCR primers specific of the internal control proximity probes. The results, given in Ct, corresponded to the mean of three duplicated experiments.

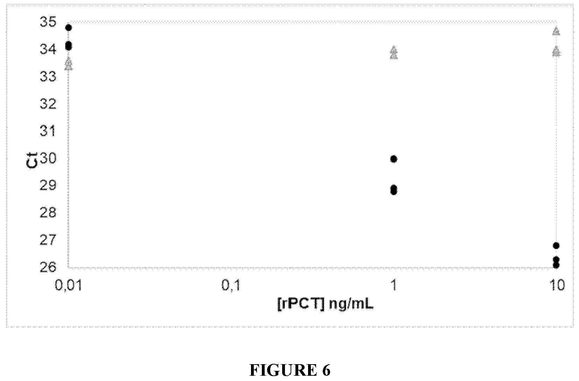

**Figure 6****:** Procalcitonin Proximity extension assay (red dots) and Internal Control Proximity extension assay (green triangles) implemented on three human plasma specimens after spiking of different concentrations of rPCT (From 0.01 to 10 ng/mL) plus 50 ng/mL of Bitag 2. The results, given in Ct, corresponded to the mean of duplicated experiments.

### EXAMPLES

### Example 1: Synthesis of Bitag-1 et Bitag-2

The synthetic peptide syntheses were carried out using either the ABI 433A synthesizer from Applied Biosystems (Foster City, Calif., United States). The Rink Amide MBHA resin (Cat. No. 855003, Novabiochem^{®}, Merck Millipore, Molsheim, France) was used as polymeric solid support. At the end of the chemical synthesis, the peptides were deprotected and cleaved from the polymer in the presence of a mixture of trifluoroacetic acid-ethanedithiol-triisopropylsilane-water (94/2.5/1/2.5 V/V/V/V) for approximately 2 hours. After elimination of the polymer by filtration, the peptides were isolated by precipitation from diethyl ether at 0°C. In order to increase their degree of purity, the peptides were purified by reversephase preparative high performance liquid chromatography (HPLC) on a Vynac Denali^{™} 120 Å C18, 10 µm column (Mandel Scientific Company Inc., Guelph, Ontario, Canada). Each peptide was eluted with a stepwise gradient of acetonitrile (from 0 to 95%) in aqueous solution containing 0.1% of trifluoroacetic acid, the percentage of acetonitrile of the steps having been chosen so as to optimize the isolation of the peak which corresponds to the peptide of interest. After this final step, two different analysis techniques were carried out in order to verify and characterize the peptides obtained.

### Example 2: Development of a bi-epitope peptide as fail-safe internal control for proximity extension assay and implementation in human plasma specimens

### Material and Methods

**Material.** Recombinant human procalcitonin (rPCT) was developed and produced by bioMérieux SA (Lyon, France) after protein expression in prokaryotic cells, according to standard procedures [8]. rPCT was stored at -80°C in a PBS buffer containing 5% bovine serum albumin (BSA).

Monoclonal antibodies were generated and purified by bioMerieux SA according to standard procedures after mice immunizations with rPCT or with peptides [9]. rPCT antibodies were either directed against the calcitonin domain of the procalcitonin (8F12 monoclonal antibody) or the katacalcin domain of the PCT (11E12 monoclonal antibody) and are non-commercial antibodies. Anti-tag antibodies were directed either against a 6xHistidine tag (MRGSHHHHHH) or a 6xLysine tag (GKKKKKKSV) and were respectively called 18D12 or 2G4A12/5F12E5 [10]. Peptides were internally synthesized using Fmoc chemistry on a ABI433A peptide synthesizer from Applied Biosystems following the manufacturer's instructions (Foster City, CA USA).

Whole blood specimens were obtained from healthy donors from the French National Blood Bank (EFS, Etablissement Français du Sang, Lyon, France). Samples were selected on the basis of a negative PCT test. Fresh blood was centrifuged for five min at 20 000 g, at 4°C, using a refrigerated microcentrifuge (Eppendorf 5424R, Montesson, France). After centrifugation, the supernatant, which corresponds to the plasma, was recovered for Proximity extension assay and stored at 4°C before use. For all biological samples, informed consent was obtained for any experimentation. All experiments were performed in compliance with the relevant laws and institutional guidelines and in accordance with the ethical standards of the Declaration of Helsinki.

**Proximity probes.** Proximity probes were prepared by covalently linking purified PCT monoclonal antibodies to HPLC-purified oligonucleotides. Oligonucleotides had a C6-amin modification at their 5'-end and contained, from 5'- to 3'-end, a 18-22 nucleotides sequence for PCR primer hybridization and a 9 nucleotides sequence for hybridization with the second proximity probe. Synthesis, modifications and HPLC-purification of the oligonucleotides were all performed by Integrated DNA Technologies (Louvain, Belgium). Antibody-oligonucleotide conjugates were generated using commercial Thunderlink kit (Abcam, Cambridge, UK), following the manufacturer's instructions, using a 5:1 oligonucleotide-to-antibody ratio. Conjugation quality was checked by running conjugates on a reducing 4-12% SDS-PAGE (Thermofisher, Waltham, MA, USA) followed by Coomassie Blue staining (Thermofisher) as recommended by the supplier's instructions (Abcam).

The sequences of the two pairs of nucleotides (one pair for PCT proximity probes, the other pair for internal control proximity probes), were generated randomly (https://molbiotools.com), taking care to avoid homeo-tracks and palindromic sequences. The PCR primers sequences have a Tm around 60°C.

**Enzyme Linked Immuno-Sorbant Assays (ELISA).** After overnight sensitization with peptides diluted at 5 µg/mL in PBS buffer, pH 7.4 (Euromedex, Souffelweyersheim, France), Nunc 96-well plates (Thermofisher) were saturated for 1 h at room temperature (RT) with PBS1x-Bovine Serum Albumin 5% (BSA, Sigma-Aldrich, Saint Louis, MI, USA). After 4 washes with PBS1x-Tween20 0.1%, PBS1x- BSA 1% buffer containing the anti-tag antibody (at 1 µg/mL) was added and incubated for 1 h at 37°C. The developing with PNPP substrates (Thermofisher) was performed after 30 min of incubation at 37°C with a 1/50 000 dilution in PBS-BSA-0.5% of an alkaline phosphatase (AP)-labeled streptavidin (Jackson Immunoresearch, Ely, UK). The absorbance level (optical density, OD) was red at 405 nm (OD405) using an Infinite M Nano+ microplate reader (Tecan, Männedorf, Switzerland). All samples were tested in duplicate and in three independent experiments.

**Proximity extension assay.** The Proximity extension assay protocol and the different buffers were taken over from the protocol initially described by Lundberg et al. [1] and also from a recently developed shorter protocol (unpublished results). Proximity extension assay was performed in specific multi-well plates (Hard-Shell 96-Well Clear shell PCR Plates, BioRad, Hercule, CA, USA), in triplicate. Briefly, 5 µL of sample (PBS1x-0.1% BSA buffer or human EDTA plasma with or without rPCT and/or IC peptide) were mixed with 5 µL of a mix of the two PEA conjugates (500 pM each) diluted in the probe incubation buffer and incubated at 37°C for 60 min. After probe incubation, a dilution buffer containing 40 mM of each dNTP were added. After a 5 minutes incubation step at 37°C, 96 µl extension mix containing 26 U/mL of T4 DNA Polymerase (Thermofisher) were added and incubated at 37°C for an additional 3 minutes, followed by a heat inactivation step at 80°C.

**Detection by qPCR.** For qPCR detection, 4 µL of extension products were transferred to a qPCR plate and mixed with 36 µL of qPCR mix (SYBRGREEN mix, Bio-Rad) containing 0.6 µM of each PCR primer. A one-step qPCR was run with initial denaturation at 95°C for 3 min, followed by 1 sec of denaturation at 95°C and 10 sec annealing/extension at 63°C for 40 cycles. Finally, a melt step was performed by increasing the temperature gradually from 60°C to 95°C at 0.5°C/sec.

**Statistical analysis.** Statistical data analysis was performed using GraphPad Prism v4.03 software (GraphPad Software, San Diego, CA, USA). Comparisons of continuous variables were performed using Mann-Whitney U test (that can be applied on unknown distributions for two small sets of observations, n < 30). Histograms were drawn using Excel, confidence intervals (error bars) correspond to 2 times the standard deviation.

### RESULTS

**Internal control design selection.** One key consideration in the design of the internal control was the need to prevent interferences from proteins naturally present in human blood products. After considering using a non-human protein as an internal control, the choice was finally made to evaluate synthetic peptides containing two non- human epitopes, more specifically epitope tags. His- and Lystags have been used previously for protein purification and for functionalization of microplates for immunoassays [10, 11].

As described earlier, two epitopes are required for the binding of Proximity extension assay probes to their target. The binding of the two proximity probes (antibody conjugates) to these epitopes must not interfere with each other by steric hindrance. For this reason, the two epitopes on the internal control peptide were separated by a neutral peptide bond that acts as a spacer. The spacer was made from Ado (Amino Dioxa-octanoic Acid [12]) moieties, which consist of a linear chain of three artificial amino acids, for a total size of 10 Angstroms.

A concatenation of two or three Ado spacers was inserted between the two epitope-tags of the IC for a distance of 20 and 30 Angstroms, respectively. The peptide with two Ado spacers was called Bitag 1, while the peptide with 3 Ado spacers was called Bitag 2.

**Selection and validation of the Bitag peptides and the proximity probes.** First, to check that the anti-tag antibodies do bind to their epitopes in the context of the Bitag peptides, indirect ELISA was performed by sensitizing multiwall plates with either Bitag 1 or Bitag 2 peptides at two concentrations (1 µg/mL and 5 µg/mL). For the two coated peptides, 18D12 monoclonal antibody (anti-His tag) or 2G4 and 5F12 monoclonal antibodies (anti-Lys tag) were used for detection with an AP-labelled anti-mouse conjugate.

Figure 1 shows that without peptide, a small signal (OD405< 0.126) was observed. This signal was 5.42 times (18D12 antibody, 1µg of Bitag 1) and 9.17 times (2G4 antibody, 5 µg of Bitag 1) lower than the signal obtained in the presence of peptide, confirming that antibodies did bind specifically to the peptide. In addition, a small signal (between 0.124 and 0.138) was obtained without anti-tag antibody, which reflected weak non-specific binding of the AP-conjugate. Overall, all the antibodies did bind to the two Bitag peptides. The signal observed for 18D12 (anti-His) was lower that the signal obtained with the anti-tag Lys antibodies (2G4 and 5F12). For instance, the OD405 for 1 µg of Bitag 1 peptide was 0.617 for 18D12, while it was 0.993 for 2G4. For 5 µg of peptide, OD405 increased significantly for 18D12 (+ 0.262) whilst there was a moderate increase for 2G4 and 5F12 (+ 0.089 and + 0.074, respectively). No significant difference was observed between the two anti-Lys tag antibodies 2G4 and 5F12. Nevertheless, because a slight advantage in terms of OD value was systematically observed, 2G4 was preferred over 5F12 in subsequent experiments.

Consequently, the two monoclonal antibodies 18D12 (anti-His tag) and 2G4 (anti-Lys tag) were used in the preparation of the internal control proximity probes.

Second, to determine the best combination of spacer size and oligonucleotide length, proximity probes were tested successively on the two Bitag 1 and Bitag 2 peptides. The first type of probes, called MML, featured a longer version of the oligonucleotides (49 and 56 bases), whilst the second type of probes, called MMC, featured a shorter version of the oligonucleotides (29 and 31 bases). For both MML and MMC, the size of the complementary sequence located at the 3'-end of each oligonucleotide was 9 nucleotides.

Figure 2 summarizes the proximity extension assay results obtained for three concentrations of Bitag peptide (0, 5 and 50 ng/mL) spiked in PBS-BSA buffer. The results are expressed in Delta-Ct0-X, corresponding to the difference in Ct values between 0 and X ng/mL of peptide. Diagrams in Figure 2 show the significant gain in Delta-Ct observed for MMC when compared with MML probes. For instance, for Bitag 2, the gain for MMC versus MML was 2.32 for 5 ng/mL of peptide and 1.86 for 50 ng/mL. For MMC, Delta- Ct0-50 reached 7.08 (versus 5.22 for MML). The Delta-Ct for MMC was also found to be higher for Bitag 1, but to a lesser degree.

The MMC probes were the most suitable for proximity extension assay, whatever the peptide tested. The combination of the shorter MMC oligonucleotides with the peptide featuring a spacer containing 3 Ado sequences (Bitag 2) gave the highest Delta-Ct, for the peptide concentrations tested.

In order to confirm these preliminary results obtained on peptide diluted in the PBS-BSA buffer, PEA was performed, using MMC probes, on human plasma (pool of three plasma specimens) spiked with four concentrations of Bitag 2 peptide, from 0 to 500 ng/mL. The results are illustrated in Figure 3a. Overall, while the assay presented a similar dynamic to that obtained in PBS-BSA buffer, Delta-Ct values appeared to be slightly lower compared to those obtained in PBS-BSA (loss of 0.26 and 0.36 for 5 ng/mL and 50 ng/mL of peptide, respectively). In addition, for 500 ng/mL, a significant loss of dynamic was observed (Delta-Ct50-500 of 0.9 versus Delta-Ct5-50 of 2.72).

Taken together, these results showed that it was possible to detect a peptide having two epitope-tags by using a fast Proximity extension assay protocol in a complex medium (pool of human plasma samples).

Third, a Bitag 2 concentration suitable for internal control Proximity extension assay had to be chosen. 50 ng/mL was selected because it provided a signal level (Ct value) significantly above the background, but still in the dynamic range. To confirm the specificity of the results obtained with 50 ng/mL of Bitag 2 peptide, human plasma specimens were tested by Proximity extension assay after addition of 50 ng/mL of peptide (n= 25 samples) or without peptide complementation (n= 13 samples). The results obtained are summarized in Figure 3b. According to a Mann-Whitney test, the difference between the two groups was significant (P<0.001).

**Interference between procalcitonin and internal control.** In order to check that the Bitag peptide did not interfere with the detection of procalcitonin, the Proximity extension assay protocol for procalcitonin detection was performed in PBS-BSA buffer spiked with two concentrations of rPCT (0 and 0.1 ng/mL) and six concentrations of Bitag 2 peptide (from 0 to 200 ng/mL). The results are illustrated in Figure 4. For 0.1 ng/mL of rPCT, no significant difference was observed across the range of peptide concentrations added to the sample (mean = 34.38 ± 0.15). Similar results were obtained for 0 ng/mL of rPCT, except for 160 ng/mL of peptide where a significantly lower Ct was observed (35.9, for an average value of 36.98 for the other peptide concentrations).

in order to check that PCR primers used for qPCR amplification of procalcitonin products did not interfere with primers used for Internal control proximity probes, the Proximity extension assay protocol for procalcitonin detection was performed in PBS-BSA buffer containing the two analytes, i.e. rPCT (at 0 and 0.1 ng/mL) and Bitag peptide (at a range of concentrations from 0 to 160 ng/mL). Both analytes were subjected to the Proximity extension assay protocol in parallel, but PCR amplification was conducted in presence of either the primers specific of the procalcitonin PEA probes or the primers specific to the Internal control proximity probes. As showed in Figure 5a, no significant difference in Ct values was observed for the different concentrations of Bitag peptide when the PCT primer set was used. For 0.1 ng/mL of rPCT, the average value was 34,26 ± 0.31. For 0 ng/mL of PCT, a slight but significant increase in background noise was observed after addition of 40 ng of peptide with respect to a sample without peptide. The Delta-Ct between these two conditions was 1.4. Interestingly, the Ct observed for 40 ng/mL of peptide was the same as that observed with 160 ng/mL of peptide.

When the internal control PCR primer set was used for PCR amplification (see Figure 6b), no interference was observed in the presence of procalcitonin. Ct values obtained for each peptide concentration were not significantly different whether procalcitonin was present (at 0.1 ng/mL) or not.

Both proximity extension assay protocols can therefore be performed without significant inhibiting effects on their PCR amplification performance.

**Implementation in human plasma specimen.** Human plasma contains numerous endogenous potentially interfering substances, such as antibodies possessing broad reactivity toward mouse antibodies, that may hinder proximity extension assay performance [13]. In order to confirm that it was possible to detect procalcitonin and internal control simultaneously in human samples, three plasma specimens taken from three different patients were spiked both with rPCT at different concentrations (0, 1 and 10 ng/mL) and with the Bitag peptide at 50 ng/mL. As illustrated in Figure 6, it was possible to detect rPCT at concentrations as low as 1 ng/mL in human plasma. As expected, the Ct declined when the concentration of procalcitonin increased. The average Delta-Ct observed between 0 and 1 ng/mL of rPCT was 5.2. Between 1 and 10 ng/mL, the average Delta-Ct decreased to 2.8.

The Ct values for the internal control did not vary significantly with the three rPCT concentrations and across the different patients. The Ct mean value was 33.53 ± 0.11 for 0 ng/mL of PCT and 34.23 ± 0.40 for 10 ng/mL of PCT.

All the results obtained with Bitag 2 peptide have also been reproduced and confirmed with Bitag 2' peptide (data not shown).

### DISCUSSION

In brief, it has been shown that a peptide made up of two epitope-tags can serve as a fail-safe internal control in immunomolecular assay such as proximity extension assay. The internal control (synthetic peptide of the disclosure) can be simultaneously detected with at least one biomarker of diagnostic interest in human plasma specimens, without cross-interference between the control and the analyte and with no evidence of major interference with plasma proteins. The combination of the shorter MMC oligonucleotides with the peptide featuring a spacer containing 3 Ado sequences gave the best performance using a proximity extension assay protocol as proof of concept.

In terms of design and implementation of an internal control for an immunomolecular assay, it was first necessary to ensure that the internal control could be generated with sufficient batch to batch reproducibility through a wellcontrolled synthesis process. To this end, it was deemed preferable to work with a recombinant protein or a synthetic peptide, as opposed to a naturally occurring molecule. Second, this protein or peptide could not be naturally present in human samples, as the signal associated with the internal control must be always the same, regardless of the sample tested. In addition, the internal control had to feature two distinct epitopes for which two specific monoclonal antibodies had to be available (one antibody per epitope, at least) to serve as probes.

Each epitope should ideally be mapped so as to know its precise location on a 3D structure (when available) of the protein analyte. In order to facilitate production and also to avoid limiting the accessibility of epitopes on the surface of the protein, it was deemed preferable to select a non-glycosylated protein. Finally, the epitopes had to be suitable for the proximity extension or ligation assay approach, in terms of proximity and accessibility on the protein or peptide. Indeed, if the epitopes are buried inside the protein or too far away from each other, this can have a negative impact on the development of a successful proximity extension assay protocol. With these considerations in mind, it was decided to use a synthetic peptide, rather than a non-human recombinant protein (e.g. from plant, invertebrate), as the IC.

In previous studies, Arrarsson et al. [14] elected to use a spike-in control made of a mix of different recombinant proteins, including Green Fluorescent Protein (GFP) and phyco-erythrin. These controls were used for monitoring the incubation step of proximity assay, for identifying possible outliers among the samples and also for performing inter-plates normalization. Arrarsson et al. also used an extension control consisting of an antibody linked to oligonucleotides. Additionally, a control for qPCR was made from double-stranded DNA template. These controls were shown to be useful to improving both intra-assay (for multiplexing) and inter-assay precision by compensating for technical variations.

Epitope-tags are short peptides (3-14 amino acids) typically used either to facilitate protein purification or to monitor protein localization, movement, modification, or interactions during physiological processes [15, 16]. Epitope-tags have the ability to be recognized and bound by a tag-specific antibody. A wide range of epitope tags have traditionally been developed and used. They have been selected to not interfere with cellular function or processing. They are also weakly antigenic so as not to bind to host antibodies. Tags can be placed at the N-terminus, C-terminus or in the reading frame of the protein to be studied. They are usually derived from protein sequences of viruses and phages, such as the peptide tag of herpes simplex virus glycoprotein D or the peptide tag of phage T7 capsid protein [17, 18].

Here, it was decided to assess peptides comprised of two epitope-tags (His-tag and Lys-tag) separated by a neutral spacer consisting of three concatenated Ado bridges. Indeed, the process of synthesis of these peptides were strictly controlled and the distance between the two tags was determined by the number of Ado bridges. Moreover, by nature, a peptide is poorly structured and the possibility for the epitopes to be not accessible to antibodies is low. Finally, several monoclonal antibodies directed against each epitope-tag of the peptide were available.

One of the frequent problems with the use of linear peptides is their lack of stability [19] [20]. For long term storage, peptides are typically kept at -20°C. However, it was demonstrated that peptides presented in this present disclosure were stable at least twelve weeks (86 days) at 4°C, and at least 24 h at room temperature (data not shown). It is therefore possible to use these peptides under common storage conditions and over a prolonged period of time with limited risk of significant degradation.

In an effort to determine the best trade-off between the size of the peptide on the one hand, and the length of the oligonucleotides linked to the antibodies on the other hand, it was demonstrated that, at the scale of the peptide, the oligonucleotides had to be as short as possible to bring a significant gain in terms of assays sensitivity.

Using these conditions, the internal control could be detected by immunomolecular assay, for example by proximity extension or ligation assay, over a range of concentrations corresponding to a dynamic range of about 3 orders of magnitude. Moreover, the impact of peptide concentration on procalcitonin proximity extension assay performance was limited and no interference between the two systems of qPCR amplification was observed.

When tested in parallel with procalcitonin on several human samples, Ct values for the internal control remained stable while the Ct values for procalcitonin decreased with increasing procalcitonin concentrations, as expected.

Also, the internal control was primarily intended as a fail-safe control for the entire proximity extension assay protocol, including qPCR. As such, the internal control was designed to provide a qPCR signal (Ct value) that had to fall within a predetermined acceptance range for the assay to be considered valid. However, the internal control might be amenable to be used as a calibrator, whereby the signal obtained from the internal control is used to normalize the signal from the analyte.

In the present disclosure, proximity extension assay was performed in multiwell plates, with the internal control added at the first step of the proximity extension assay protocol.. The internal control could also be implemented in an automated version of the proximity extension/ligation assay protocol, most interestingly as part of a system intended for diagnostic applications, for example in the context of a point of care solution.

### Example 3: Description of the nucleotide sequences for use in the present disclosure

| SEQ ID NO: | Description of the sequence |
|---|---|
| 1 | His tag peptide |
| 2 | Long His tag peptide |
| 3 | Lys tag peptide |
| 4 | Long Lys tag peptide |
| 5 | Bitag-1 |
| 6 | Bitag-2 |
| 7 | Bitag-1' |
| 8 | Bitag-2' |
| 9 | Full oligo 1 sequence of bitag 2 MMC |
| 10 | Full oligo 2 sequence of bitag 2 MMC |

| SEQ ID NO: | Nucleotide (nt) or amino acid (aa) | SEQUENCE |
|---|---|---|
| 1 | aa | MRGSHHHHHH |
| 2 | aa | MRGSHHHHHHSVDES |
| 3 | aa | GKKKKKKSV |
| 4 | aa | GKKKKKKSVDES |
| 5 | aa | MRGSHHHHHHNNGKKKKKKSV |
| | | N is 8-amino-2,4-dioxa octanoic acid |
| 6 | aa | MRGSHHHHHHNNNGKKKKKKSV |
| | | N is 8-amino-2,4-dioxa octanoic acid |
| 7 | aa | MRGSHHHHHHSVDESNNGKKKKKKSVDESL |
| | | N is 8-amino-2,4-dioxa octanoic acid |
| 8 | aa | MRGSHHHHHHSVDESNNNGKKKKKKSVDESL |
| | | N is 8-amino-2,4-dioxa octanoic acid |
| 9 | nt | TAATAGTATCGAGCGGTCAGAGACGACTTC |
| 10 | nt | TAGCTAAGTGGCAGGATGAGAAGTCGTC |

### REFERENCES

1. Lundberg M, Eriksson A, Tran B, Assarsson E, Fredriksson S. Homogeneous antibody-based proximity extension assays provide sensitive and specific detection of low-abundant proteins in human blood. Nucleic Acids Res. 2011;39(15):e102. Epub 2011/06/08. doi: 10.1093/nar/gkr424. PubMed PMID: 21646338; PubMed Central PMCID: PMCPMC3159481.
2. Fredriksson S, Gullberg M, Jarvius J, Olsson C, Pietras K, Gústafsdóttir SM, et al. Protein detection using proximity-dependent DNA ligation assays. Nat Biotechnol. 2002;20(5):473-7. Epub 2002/05/01. doi: 10.1038/nbt0502-473. PubMed PMID: 11981560.
3. Greenwood C, Ruff D, Kirvell S, Johnson G, Dhillon HS, Bustin SA. Proximity assays for sensitive quantification of proteins. Biomol Detect Quantif. 2015;4:10-6. Epub 2016/04/15. doi: 10.1016/j.bdq.2015.04.002. PubMed PMID: 27077033; PubMed Central PMCID: PMCPMC4822221.
4. Schuetz P, Albrich W, Mueller B. Procalcitonin for diagnosis of infection and guide to antibiotic decisions: past, present and future. BMC Med. 2011;9:107. Epub 2011/09/23. doi: 10.1186/1741-7015-9-107. PubMed PMID: 21936959; PubMed Central PMCID: PMCPMC3186747.
5. Wacker C, Prkno A, Brunkhorst FM, Schlattmann P. Procalcitonin as a diagnostic marker for sepsis: a systematic review and meta-analysis. Lancet Infect Dis. 2013;13(5):426-35. Epub 2013/02/05. doi: 10.1016/s1473-3099(12)70323-7. PubMed PMID: 23375419.
6. Hamade B, Huang DT. Procalcitonin: Where Are We Now? Crit Care Clin. 2020;36(1):23-40. Epub 2019/11/18. doi: 10.1016/j.ccc.2019.08.003. PubMed PMID: 31733680; PubMed Central PMCID: PMCPMC6866676.
7. Harbarth S, Holeckova K, Froidevaux C, Pittet D, Ricou B, Grau GE, et al. Diagnostic value of procalcitonin, interleukin-6, and interleukin-8 in critically ill patients admitted with suspected sepsis. Am J Respir Crit Care Med. 2001;164(3):396-402. Epub 2001/08/14. doi: 10.1164/ajrccm.164.3.2009052. PubMed PMID: 11500339.
8. Schendel PF. Overview of protein expression in E. coli. Curr Protoc Mol Biol. 2001;Chapter 16:Unit16.1. Epub 2008/02/12. doi: 10.1002/0471142727.mb1601s41. PubMed PMID: 18265122.
9. Yokoyama WM. Monoclonal antibody supernatant and ascites fluid production. Curr Protoc Immunol. 2001;Chapter 2:Unit 2.6. Epub 2008/04/25. doi: 10.1002/0471142735.im0206s40. PubMed PMID: 18432770.
10. Ladavière C, Delair T, Domard A, Novelli-Rousseau A, Mandrand B, Mallet F. Covalent immobilization of proteins onto (Maleic anhydride-alt-methyl vinyl ether) copolymers: enhanced immobilization of recombinant proteins. Bioconjug Chem. 1998;9(6):655-61. Epub 1998/11/17. doi: 10.1021/bc970208i. PubMed PMID: 9815157.
11.Janknecht R, de Martynoff G, Lou J, Hipskind RA, Nordheim A, Stunnenberg HG. Rapid and efficient purification of native histidine-tagged protein expressed by recombinant vaccinia virus. Proc Natl Acad Sci USA. 1991;88(20):8972-6. Epub 1991/10/15. doi: 10.1073/pnas.88.20.8972. PubMed PMID: 1924358; PubMed Central PMCID: PMCPMC52633.
12. Basak S, Mohottalage D, Basak A. Multibranch and pseudopeptide approach for design of novel inhibitors of subtilisin kexin isozyme-1. Protein Pept Lett. 2006;13(9):863-76. Epub 2006/11/15. doi: 10.2174/092986606778256199. PubMed PMID: 17100641.
13. Klee GG. Human anti-mouse antibodies. Arch Pathol Lab Med. 2000;124(6):921-3. Epub 2000/06/03. doi: 10.5858/2000-124-0921-hama. PubMed PMID: 10835540.
14. Assarsson E, Lundberg M, Holmquist G, Björkesten J, Thorsen SB, Ekman D, et al. Homogenous 96-plex PEA immunoassay exhibiting high sensitivity, specificity, and excellent scalability. PLoS One. 2014;9(4):e95192. Epub 2014/04/24. doi: 10.1371/journal.pone.0095192. PubMed PMID: 24755770; PubMed Central PMCID: PMCPMC3995906 commercializing the described method under the name Proseek Multiplex. Patent name: "Exonuclease enabled proximity extension assays" and number: WO2012104261A1. There are no new patents, products in development or marketed products to declare. This does not alter our adherence to all the PLOS ONE policies on sharing data and materials.
15. Kolodziej PA, Young RA. Epitope tagging and protein surveillance. Methods Enzymol. 1991;194:508-19. Epub 1991/01/01. doi: 10.1016/0076-6879(91)94038-e. PubMed PMID: 1706460.Cravchik A, Matus A. A novel strategy for the immunological tagging of cDNA constructs. Gene. 1993;137(1):139-43. Epub 1993/12/27. doi: 10.1016/0378-1119(93)90262-2. PubMed PMID: 7506688.
16. Hansen MK, Tams JW, Fahrenkrug J, Pedersen PA. Functional expression of rat VPAC1 receptor in Saccharomyces cerevisiae. Recept Channels. 1999;6(4):271-81. Epub 1999/07/21. PubMed PMID: 10412720.
17. Humbert N, Schürmann P, Zocchi A, Neuhaus JM, Ward TR. High-yield production and purification of recombinant T7-tag mature streptavidin in glucose-stressed E. coli. Methods Mol Biol. 2008;418:101-10. Epub 2008/02/22. doi: 10.1007/978-1-59745-579-4_9. PubMed PMID: 18287653.
18. Wearley LL. Recent progress in protein and peptide delivery by noninvasive routes. Crit Rev Ther Drug Carrier Syst. 1991;8(4):331-94. Epub 1991/01/01. PubMed PMID: 1769066.
19. Bogdanowich-Knipp SJ, Chakrabarti S, Williams TD, Dillman RK, Siahaan TJ. Solution stability of linear vs. cyclic RGD peptides. J Pept Res. 1999;53(5):530-41. Epub 1999/07/29. doi: 10.1034/j.1399-3011.1999.00052.x. PubMed PMID: 10424348.
20. Assarsson E, Lundberg M, Holmquist G, Bjo" rkesten J, Bucht Thorsen S, et al. (2014) Homogenous 96-Plex PEA Immunoassay Exhibiting High Sensitivity, Specificity, and Excellent Scalability. PLoS ONE 9(4): e95192. doi:10.1371/journal.pone.0095192.

## Claims

1. A synthetic peptide of the following formula (I)
R₁-Z-R₂ (I)
wherein
Z is a peptide spacer consisting of 1 to 3 unnatural amino acids,
R₁ and R₂ are two different peptide moieties, wherein one peptide moiety of R₁ or R₂ comprises a polylysine tag and the other peptide moiety comprises a polyhistidine tag.

2. The synthetic peptide of Claim 1, wherein the spacer Z is having a length between about 10 to about 30 Angtroms, preferably of about 20 to about 30 Angstroms.

3. The synthetic peptide of Claim 1 or 2, wherein said spacer Z is a dipeptide or tripeptide of unnatural amino acids, each unnatural amino acid having a length of about 10 Angstroms.

4. The synthetic peptide of anyone of Claims 1-3, wherein said unnatural amino acid is 8-amino-2,4-dioxa octanoic acid.

5. The synthetic peptide of anyone Claims 1-4, wherein Z is a tripeptide of 8-amino-2,4-dioxa octanoic acid.

6. The synthetic peptide of anyone Claims 1-4, wherein R₁ is a peptide of the following sequence:
X₋₁₀X₋₉X₋₈X₋₇(X₋₆)ₙ X₋₅X₋₄X₋₃X₋₂X₋₁, wherein:
- X₋₁₀ is optional or a non-polar amino acid, preferably a methionine,
- X₋₉ is optional or a basic amino acid, preferably an arginine,
- X₋₈ is optional or a non-polar amino acid, preferably a glycine,
- X₋₇ is optional or a polar amino acid, preferably a serine,
- X₋₆ is a histidine or lysine,
- X₋₅ is optional or a polar amino acid, preferably a serine,
- X₋₄ is optional or a non-polar amino acid, preferably a valine,
- X₋₃ is optional or a polar acidic amino acid, preferably an aspartic acid,
- X₋₂ is optional or a polar acidic amino acid, preferably a glutamic acid,
- X₋₁ is optional or a polar amino acid, preferably a serine, and
- n is an integer between 4 to 8, preferably equal to 6.

7. The synthetic peptide of anyone Claims 1-6, wherein R₂ is a peptide of the following sequence:
X₊₁(X₊₂)ₚX₊₃X₊₄X₊₅X₊₆X₊₇X₊₈ wherein:
- X₊₁ is optional or a non-polar amino acid, preferably a glycine,
- X₊₂ is a lysine or histidine,
- X₊₃ is optional or a polar amino acid, preferably a serine,
- X₊₄ is optional or a non-polar amino acid, preferably a valine,
- X₊₅ is optional or a polar acidic amino acid, preferably an aspartic acid,
- X₊₆ is optional or a polar acidic amino acid, preferably a glutamic acid,
- X₊₇ is optional or a polar amino acid, preferably a serine,
- X₊₈ is optional or a non-polar amino acid, preferably a leucine, and
- p is an integer between 4 to 8, preferably equal to 6.

8. The synthetic peptide of anyone of Claim 1-7, which has the following formula (II):
X₋₁₀X₋₉X₋₈X₋₇(X₋₆)ₙX₋₅X₋₄X₋₃X₋₂X₋₁(N)ₘX+₁(X₊₂)ₚX₊₃X₊₄X₊₅X₊₆X₊₇X₊₈ (II) wherein:
- X₋₁₀ is a non-polar amino acid, preferably a methionine,
- X₋₉ is a basic amino acid, preferably an arginine,
- X₋₈ is a non-polar amino acid, preferably a glycine,
- X₋₇ is a polar amino acid, preferably a serine,
- X₋₆ is histidine,
- N is an 8-amino-2,4-dioxa octanoic acid,
- X₊₁ is a non-polar amino acid, preferably a glycine,
- X₊₂ is lysine,
- X₊₃ is a polar amino acid, preferably a serine,
- X₊₄ is a non-polar amino acid, preferably a valine,
- X₋₅, X₋₄, X₋₃, X₋₂, X₋₁, X₊₅, X₊₆, X₊₇, and X₊₈ are absent,
- m is comprised from 2 to 4 preferably is equal to 3, and,
- n and p are comprised from 4 to 8, preferably are equal to 6.

9. The synthetic peptide according to claim 8, wherein:
- X₋₁₀ is a non-polar amino acid selected from glycine, alanine, valine, leucine, isoleucine, proline, phenylalanine, methionine and tryptophane,
- X₋₉ is an arginine,
- X₋₈ is a non-polar amino acid selected from glycine, alanine, valine, leucine, isoleucine, proline, phenylalanine, methionine and tryptophane,
- X₋₇ is a polar amino acid selected from serine, cysteine, threonine, tyrosine, asparagine and glutamine,
- X₋₆ is a histidine,
- X₊₁ is a non-polar amino acid selected from glycine, alanine, valine, leucine, isoleucine, proline, phenylalanine, methionine and tryptophane,
- X₊₂ is a lysine,
- X₊₃ is a polar amino acid selected from serine, cysteine, threonine, tyrosine, asparagine and glutamine,
- X₊₄ is a non-polar amino acid is selected from glycine, alanine, valine, leucine, isoleucine, proline, phenylalanine, methionine and tryptophane,
- m is equal to 2 or 3, preferably 3, and
- n and p are equal to 5 or 6.

10. The synthetic peptide according to claim 8 or 9, which has the following sequence: MRGSHHHHHH-(N)ₘ-GKKKKKKSV (SEQ ID NO:6), wherein N is 8-amino-2,4-dioxa octanoic acid and m is 3.

11. The synthetic peptide of anyone of Claim 1-7, which has the following formula (III):
X₋₁₀X₋₉X₋₈X₋₇(X₋₆)ₙX₋₅X₋₄X₋₃X₋₂X₋₁(N)ₘX₊₁(X₊₂)ₚX₊₃X₊₄X₊₅X₊₆X₊₇X₊₈ (III) wherein:
- X₋₁₀ is a non-polar amino acid selected from glycine, alanine, valine, leucine, isoleucine, proline, phenylalanine, methionine and tryptophane,
- X₋₉ is a basic amino acid,
- X₋₈ is a non-polar amino acid selected from glycine, alanine, valine, leucine, isoleucine, proline, phenylalanine, methionine and tryptophane,
- X₋₇ is a polar amino acid selected from serine, cysteine, threonine, tyrosine, asparagine and glutamine,
- X₋₆ is an histidine,
- X₋₅ is a polar amino acid selected from serine, cysteine, threonine, tyrosine, asparagine and glutamine,
- X₋₄ is a non-polar amino acid selected from glycine, alanine, valine, leucine, isoleucine, proline, phenylalanine, methionine and tryptophane,
- X₋₃ is an acidic amino acid selected from aspartic acid and glutamic acid,
- X₋₂ is an acidic amino acid selected from aspartic acid and glutamic acid,
- X₋₁ is a polar amino acid selected from serine, cysteine, threonine, tyrosine, asparagine and glutamine,
- N is an 8-amino-2,4-dioxa octanoic acid,
- X₊₁ is a non-polar amino acid selected from glycine, alanine, valine, leucine, isoleucine, proline, phenylalanine, methionine and tryptophane,
- X₊₂ is a lysine,
- X₊₃ is a polar amino acid from serine, cysteine, threonine, tyrosine, asparagine and glutamine,
- X₊₄ is a non-polar amino acid selected from glycine, alanine, valine, leucine, isoleucine, proline, phenylalanine, methionine and tryptophane,
- X₊₅ is an acidic amino acid selected from aspartic acid and glutamic acid,
- X₊₆ is an acidic amino acid selected from aspartic acid and glutamic acid,
- X₊₇ is a polar amino acid from serine, cysteine, threonine, tyrosine, asparagine and glutamine,
- X₊₈ is a non-polar amino acid selected from glycine, alanine, valine, leucine, isoleucine, proline, phenylalanine, methionine and tryptophane,
- m is comprised from 2 to 4 preferably is equal to 3, and,
- n and p are comprised from 4 to 8, preferably are equal to 6.

12. The synthetic peptide according to claim 11, which has the following sequence: MRGSHHHHHHSVDES-(N)m-GKKKKKKSVDESL (SEQ ID NO:8), wherein N is 8-amino-2,4-dioxa octanoic acid and m is 3.

13. A kit for use as an internal control or quantitative calibrator in a proximity extension assay, said kit comprising at least the following components:
- a synthetic peptide of any one of claims 1-12, and,
- at least two proximity probes, each proximity probe comprising an antigen-binding moiety coupled to a nucleic acid domain, wherein a first proximity probe comprises an anti-His tag antibody or its antigen-binding fragment thereof that binds specifically to a His tag, and, a second proximity probe comprises an anti-Lys tag antibody or its antigen-binding fragment thereof that binds specifically to a Lys tag.

14. Use of the synthetic peptide according to any one of claims 1 to 12, or a kit according to claim 13, as an internal control or quantitative calibrator in an immunoassay, preferably in an immunomolecular assay.

15. The use of claim 14, wherein the immunomolecular assay is a proximity extension assay, a proximity ligation assay or an immuno-PCR, preferably a proximity extension assay or a proximity ligation assay, and for example, a multiplexed proximity extension assay.
